Europäisches Patentamt

(19) **European Patent Office**

**Office européen des brevets**

(11) Numéro de publication: **0 011 523**
**B1**

(12) **FASCICULE DE BREVET EUROPEEN**

(45) Date de publication du fascicule du brevet: ·
14.07.82

(51) Int. Cl.³: **C 08 J 3/24,** C 08 L 89/00,
G 02 C 7/04

(21) Numéro de dépôt: 79400732.8

(22) Date de dépôt: 11.10.79

(54) **Hydrogels de polymères protéiques naturels, leur préparation et lentilles de contact souples à partir de ceux-ci.**

(30) Priorité: 18.10.78 US 952303

(43) Date de publication de la demande:
28.05.80 Bulletin 80/11

(45) Mention de la délivrance du brevet:
14.07.82 Bulletin 82/28

(84) Etats contractants désignés:
BE CH DE FR GB IT NL SE

(56) Documents cités:
**CH-A-493 859**
**DE-A-2 757 084**
**DE-C-610 933**
**FR-A-475 504**

(73) Titulaire: **ESSILOR INTERNATIONAL Cie Générale
d'Optique, 1 Rue Thomas Edison Echat 902,
F-94028 Creteil Cedex (FR)**

(72) Inventeur: **Battista, Orlando A., 3725 Fox Hollow Road,
Fort Worth Texas 76109 (US)**

(74) Mandataire: **Foldès, Georges et al, CABINET J. BONNET
THIRION 95 Bd. Beaumarchais, F-75003 Paris (FR)**

# Hydrogels de polymères protéiques naturels, leurs préparation et lentilles de contact souples à partie de ceux-ci

La présente invention concerne d'une manière générale de nouvelles formes d'hydrogels de protéines naturelles animales et végétales présentant des propriétés supérieures par rapport aux formes actuellement connues des mêmes matières. Plus particulièrement, l'invention concerne des compositions telles que des lentilles de contact souples (à jeter, éliminables ou pour pansements) et des préparations cosmétiques, pharmaceutiques et chirurgicales contenant ces nouvelles formes de protéines naturelles animales et végétales que l'on utilise en contact avec des liquides aqueux. L'invention concerne également des procédés pour préparer à partir de ces protéines naturelles des produits qui, par rapport aux produits qu'il est actuellement possible de préparer, sont supérieurs et d'utilité accrue.

Dans la présente description et les revendications on entend par hydrogel (hydrocolloïde) de polymères protéiques naturels animaux ou végétaux, un polymère protéique d'origine naturelle ayant un poids moléculaire moyen d'environ 100 000 au moins, que l'on peut faire gonfler par l'eau dans une gamme étendue de teneurs en eau comprises entre une valeur aussi faible que 30% et 1000% et plus, et qui présente des propriétés d'ajustement de la rhéologie convenant à l'utilisation finale.

Plus particulièrement, l'invention a pour objets: des lentilles de contact souples que l'on peut, si on le désire, colorer totalement ou au moins en partie avec des colorants pour protéines, ces lentilles permettant la correction des défauts optiques de l'oeil de l'utilisateur;

des lentilles de contact que l'on peut porter en continu jusqu'à ce qu'elles deviennent troubles et que l'on peut alors jeter (lentilles à jeter) et remplacer par une lentille neuve ou une paire de lentilles;

l'ajustement des propriétés des lentilles de contact, à partir des mêmes matières premières, de façon à ce qu'on puisse adapter à l'avance leurs propriétés à leur emploi comme pandements cornéens, lentilles éliminables ou sous forme d'hydrogels rigides à propriétés rhéologiques ajustées utiles pour le remplacement de l'humeur vitrée;

l'ajustement de la composition et de la nature chimique des hydrogels de protéines naturelles pour obtenir des pansements pour les brûlures et les plaies possédant des propriétés supérieures à l'état humide;

l'adaptation de cette nouvelle technologie à la production de fibres nouvelles utiles dans le domaine des textiles et le domaine médical; et une base d'hydrogel permettant la préparation de prothèses osseuses, artérielles et similaires présentant, lorsqu'elles sont saturées par un fluide aqueux, des propriétés remarquables qu'il était précédemment impossible d'obtenir.

Dans l'art on a beaucoup travaillé avec les protéines de bas poids moléculaires (telles que les gélatines animales) et les protéines végétales (telles que les protéines de soja) et on les a utilisées avec divers degrés et divers types de réticulation ou sans réticulation. Cependant les produits, en particulier les formes structurales correspondant à celles de l'invention, présentent des limitations importantes, en particulier en ce qui concerne les propriétés physiques à l'état humide, qui réduisent leurs applications. En particulier, dans l'art antérieur, on a nettement conclu que la gélatine ou la gélose ne permettraint jamais de produire des lentilles de contact (Soft Contact Lens, par Montague Ruben, John Wiley & Sons, p. 25, 1978).

L'invention concerne de nouvelles formes de polymères protéiques traités qui possèdent des propriétés physiques remarquables en particulier à l'état humide, ces propriétés dépendant de façon rigoureuse d'une séquence spécifique d'ajustement du pH, de concentration de l'agent de réticulation et surtout du séchage des structures à des valeurs critiques selon des séquences spécifiques échelonnées de déshydratation chimique et de déshydratation par solvant. Les produits préparés dans les conditions de réticulation et de séchage de l'art antérieur ne présentent pas la remarquable intégrité physique à l'état humide des produits préparés selon l'invention. Leur intégrité physique ne leur permet pas d'atteindre les mêmes performances à l'état humide que les produits de l'invention.

Pour préparer les produits de l'invention, il est essentiel d'utiliser au départ des matières premières protéiques naturelles qui forment des solutions limpides dans l'eau à des concentrations atteignant 30% ou plus. La gélatine non aromatisée à usage domestique constitue une protéine animale naturelle typique utile comme matière première dans l'invention et la protéine comestible de soja constitue un exemple typique de protéine végétale de départ.

Selon la forme désirée du produit final, l'ajustement du pH, l'agent de réticulation, la température, la concentration en matières solides et les séquences de déshydratation par solvant, constitue des variables essentielles. Une autre variable essentielle est un séchage à l'air à des températures ne dépassant pas environ 35° C jusqu'à une teneur en humidité de la forme séchée ne dépassant pas 10% et de préférence 7% ou moins. Si l'on n'effectue pas ces stades essentiels, par exemple si l'on chauffe le produit à 40° C ou plus en un moment quelconque avant le séchage final à l'air à une teneur en humidité ne dépassant pas 10% et qu'on n'effectue pas les traitements appropriés de déshydratation avec un solvant organique, les produits finals présentent une instabilité à l'eau inacceptable. Au lieu d'être extrêmement souples et flexibles et de présenter une bonne intégrité physique, en particulier des propriétés supérieures de résistance au déchirement à l'état humide, les produits que l'on sèche de quelque façon avant de les avoir séchés à l'air jusqu'à une humidité ne dépassant pas 10% et de préférence inférieure à 7%, avant de les soumettre à des traitements de déshydratation avec un solvant organique, ont une structure caséeuse et une forme physique extrêmement cassante et friable lorsqu'ils sont saturés d'eau. Bien entendu, cette absence d'intégrité physique à l'état humide, qui est très caractéristique des produits de l'art antérieur, rend ces produits essentiellement inutilisables pour la préparation des produits qui font l'objet de l'invention, par exemple lentilles de contact souples, pansements ophtalmologiques, cornées artificielles, humeurs vitrées, nouvelles fibres protéiques utiles dans le domaine des textiles et le domaine chirurgical, prothèses telles que cartilages et os artificiels, capsules, matériaux de suture, pansements pour les brûlures et les plaies, etc. Une des caractéristiques importantes de l'invention est qu'elle permet de préparer une gamme étendue de produits ayant un ensemble important de propriétés utiles à partir de matières premières protéiques naturelles de poids moléculaire relativement faible, peu coûteuses et abondantes.

Bien que dans les exemples on utilise la formaline (solution à 37% de formaldéhyde) comme agent de réticulation, on peut utiliser d'autres agents de réticulation appropriés tels que le glyoxal, le glutaraldéhyde, le diméthacrylate d'éthylèneglycol, le diméthacrylate de diéthylèneglycol, ou le méthylène bis-méthacrylamide, avec une solution aqueuse, par exemple une solution à 4% en poids de persulfate d'ammonium ou de persulfate de potassium, ou un autre amorceur peroxydique de polymérisation et un activateur, par exemple le diméthylènamino-2 acétate de méthyle, ou un acide p-toluènesulfonique. On peut ajouter une petite quantité de sel cuivré ou de sel de fer divalent.

On prépare les produits de l'invention à partir de solutions de protéine naturelle contenant environ 0,5% à environ 15% en poids et de préférence 0,5% à 10% en poids de la protéine ou de mélanges de protéines. On chauffe la solution à 60±5°C de façon à favoriser la dissolution de la protéine et obtenir une solution limpide. Après la dissolution de la protéine, on ajuste le pH de la solution entre environ 3,5 et environ 5,5 pour former une solution aqueuse acide de la protéine. On maintient la solution acide dans cet intervalle des températures et on ajoute à la solution un agent de réticulation qu'on lui incorpore par mélange énergique. La formaline du commerce qui contient 37% de formaldéhyde constitue un agent de réticulation particulièrement pratique et satisfaisant. On ajoute la formaline à la solution en une quantité suffisante pour obtenir environ 0,5% à environ 15%, de préférence 0,5% à 10%, de formaldéhyde par rapport au poids de la protéine. On peut remplacer la formaline par des quantités équivalentes d'autres agents de réticulation.

On coule la solution ou on la façonne à la configuration ou à la structure tridimensionelle désirée puis on sèche à l'air à une température ne dépassant pas 35°C jusqu'à une teneur en humidité ne dépassant pas 10% et de préférence inférieure à 7%. Lorsqu'on désire un produit limpide comme l'eau, on blanchit la structure séchée avec un agent oxydant tel que par exemple le peroxyde d'hydrogène, l'hypochlorite de sodium et similaires. Lorsque la couleur jaune brun caractéristique des protéines de bas poids moléculaire n'est pas gênante, on peut supprimer ce traitement. On lave à fond avec de l'eau la structure blanchie ou non blanchie puis on la soumet à un traitement de déshydratation par immersion dans un solvant organique miscible à l'eau tel que par exemple l'éthanol, l'éthanol dénaturé, l'isopropanol, l'acétone et similaires. Après le traitement par le solvant organique, on lave à fond à l'eau la structure et on la sèche à l'air à des températures ne dépassant pas 35°C pour abaisser la teneur en humidité à une valeur ne dépassant pas 10% et de préférence inférieure à 7%.

Comme la matière première de base est protéique, les produits préparés selon l'invention se prêtent à une teinture permanente unie ou multicolore éventuellement avec des motifs selon les techniques de teinture couramment utilisées pour les matières polymères contenant des radicaux NH, $NH_2$ et COOH. Par conséquent, on peut conférer à ces produits par addition de colorants ou de pigments appropriés une couleur désirée quelconque pour des raisons fonctionnelles ou esthétiques.

Les exemples non limitatifs suivants illustrent des modes de réalisation préférés de l'invention.

### Exemple 1

On chauffe à 60°C, 400 ml d'un gel limpide constitué de 400 ml d'une solution à 10% de gélatine (ayant un poids moléculaire d'environ 50 000 et une opalescence (en anglais : bloom) de 225). On ajoute à ce mélange limpide que l'on agite doucement et régulièrement, 4 gouttes d'acide chlorhydrique 10 N pour porter le pH à 4,3. Immédiatement après, on ajoute 8 ml de formaline soit environ 7,5% de formaldéhyde par rapport à la gélatine, en agitant énergiquement et en maintenant la température à 60±5°C.

On doit effectuer la coulée initiale de l'hydrogel naturel préconditionné comme ci-dessus pour produire un produit stable vis-à-vis de l'eau, rapidement après l'addition de l'agent de réticulation. On peut effectuer la coulée par séchage au repos dans l'air dans un moule ou par coulée centrifuge et séchage dans un moule.

## Lentilles de contact souples à jeter

Avec une burette ou une pipette calibrée, on introduit 4 gouttes du mélange ci-dessus dans des moules standards en polyméthacrylate de 11 mm de diamètre dans les conditions de température ordinaire. L'épaisseur de la lentille de contact ainsi que ses propriétés de réfraction sont déterminées par les caractéristiques de concavité de ces moules et par le nombre de gouttes qu'on y introduit à une concentration donnée. On laisse l'hydrogel sécher lentement à la température ordinaire (environ 25° C) pendant au moins 24 heures pendant lesquelles la réaction de réticulation s'effectue essentiellement jusqu'à son achèvement.

A la fin de ce cycle de séchage, on soumet les lentilles alors qu'elles sont encore dans leurs moules à un séchage complémentaire dans une étuve à circulation d'air pendant au minimum 4 heures à une température maximale de 30—35° C ou à une température équivalente sous vide pour être certain qu'à ce moment de la production on a abaissé la teneur en humidité à une valeur ne dépassant pas 10% et de préférence inférieure à 7%.

On retire alors facilement les lentilles des moules et on les plonge immédiatement dans une solution aqueuse diluée de peroxyde d'hydrogène ordinaire pour usage domestique (3% en volume) pendant une heure au minimum à la température ordinaire (25° C). Ce stade est particulièrement efficace pour éliminer la couleur jaune brun ou brun clair caractéristique des protéines naturelles de bas poids moléculaire qu'elles soient réticulées ou non.

On soumet ensuite les lentilles à un lavage énergique et complet avec de l'eau distillée de préférence, pour assurer l'élimination totale des petites quantités résiduelles d'agent de réticulation et de peroxyde d'hydrogène.

Après ce traitement de lavage poussé, on introduit les lentilles gonflées dans l'eau dans de l'alcool éthylique du commerce (95% en volume) dénaturé ou non où on les laisse séjourner pendant au minimum 2 heures.

A la fin du traitement de déshydratation par trempage dans l'alcool éthylique, on lave à nouveau à fond les lentilles avec de l'eau distillée pour éliminer toute trace d'alcool éthylique résiduel et des composants de dénaturation dans le cas où on utilise de l'alcool éthylique dénaturé. La répétition des lavages poussés dans de l'eau distillée de préférence, est importante à ce stade.

On sèche alors lentement à l'air les lentilles limpides que l'on vient de relaver à fond selon la séquence précise précédemment décrite qui consiste à sécher à l'air pendant 24 heures au voisinage de la température ordinaire (25° C) puis pendant au minimum 4 heures dans une étuve à circulation d'air à une température maximale de 30 à 35° C, jusqu'à ce que la teneur résiduelle en humidité ne dépasse pas 10% et de préférence soit inférieure à 7%. Si on le désire, on peut sécher les lentilles ayant reçu le dernier lavage à l'eau dans les moules où on les a coulées au départ, bien que cette façon de procéder soit facultative. On peut également, si on le désire, effectuer une stérilisation par chauffage à sec à des températures atteignant 120° C.

La séquence de fabrication s'achève ainsi (on effectue ensuite un examen, un biseautage éventuel des bords, un conditionnement et une stérilisation, etc.) pour obtenir des lentilles de contact souples, limpides comme l'eau, ayant des propriétés physiques et des propriétés de transmission des gaz remarquables à l'état humide comme à l'état sec. Parmi ces avantages, on peut citer une hydratation presque immédiate dans l'eau (2 à 3 minutes au lieu de 1 à 2 heures ou plus dans le cas des lentilles de contact souples en polymère synthétique de type HEMA) ainsi que des valeurs élevées de la perméabilité à l'oxygène gazeux.

### Exemple 2

On chauffe à 60° C 400 ml d'un gel limpide constitué de 300 ml d'une solution à 10% de gélatine et 100 ml d'un collagène comestible de boeuf partiellement hydrolysé (poids moléculaire environ 100 000) à 1%. On ajoute à ce mélange tandis que l'on agite de façon douce et régulière, 4 gouttes d'acide chlorhydrique 10 N pour porter le pH à 4,65. Immédiatement après, on ajoute au mélange énergiquement agité, 8 ml de formaline soit environ 9,5% de formaldéhyde par rapport à la protéine, en maintenant la température à 60 ± 5° C.

On doit effectuer la coulée initiale de l'hydrogel naturel préconditionné comme ci-dessus pour obtenir un produit stable à l'eau, rapidement après l'addition de l'agent de réticulation.

### Lentilles de contact souples classiques

Avec une burette ou une pipette calibrée, on introduit avec précaution 4 gouttes du mélange ci-dessus dans des moules de polyméthacrylate standards de 11 mm de diamètre dans les conditions de température ordinaire. L'épaisseur de la lentille de contact ainsi que ses propriétés de réfraction sont déterminées par les caractéristiques de concavité des moules et le nombre de gouttes introduites à une concentration déterminée.

On effectue le séchage à l'air, le blanchiment, le lavage, la déshydratation, le lavage et le séchage final à l'air comme décrit en détail dans l'exemple 1.

On obtient des lentilles de contact limpides comme l'eau qui constituent des lentilles de contact souples pouvant être portées en continu. On peut produire ces lentilles en série de façon économique, si bien que l'on peut les employer comme lentilles que l'on jette lorsqu'elles présentent un trouble excessif dû à des dépôts, etc. Si on le désire ces lentilles de contact permettent de se passer de l'emploi répété des solutions coûteuses de nettoyage et de supprimer les risques d'infection liés aux manipulations auxquelles on les soumet chaque jour lorsqu'on les retire pour les nettoyer.

### Exemple 3

On chauffe à 60°C 400 ml d'un gel limpide constitué de 400 ml d'une solution de gélatine à 10% (ayant un poids moléculaire d'environ 50 000 et une opalescence de 225). On ajoute à ce mélange limpide en agitant de façon douce et régulière, 4 gouttes d'acide chlorhydrique 10 N pour porter le pH à 4,76. Immédiatement après on ajoute au mélange en agitant énergiquement, 80 gouttes (4 ml) de formaline soit environ 3,7% de formaldéhyde par rapport à la gélatine, en maintenant la température à 60±5°C.

On doit effectuer la coulée initiale de l'hydrogel naturel préconditionné comme ci-dessus pour préparer un produit stable à l'eau, rapidement après l'addition de l'agent de réticulation.

### Lentilles de contact souples constituant des pansements éliminables

Avec une burette ou une pipette calibrée, on introduit avec précaution 4 gouttes du mélange ci-dessus dans des moules de verre spéciaux de 14 à 15 mm de diamètre dans les conditions de température ordinaire. L'épaisseur des lentilles de contact ainsi que leurs propriétés de réfraction dépendent des caractéristiques de concavité des moules et du nombre de gouttes ajoutées à une concentration donnée.

On effectue le séchage à l'air, le blanchiment, le lavage, la déshydratation, le lavage et le séchage final à l'air comme décrit en détail dans l'exemple 1.

On achève ainsi la séquence de fabrication (on effectue ensuite une inspection, un biseautage éventuel des bords, un conditionnement, une stérilisation, etc.) de lentilles de contact souples limpides comme l'eau qui possèdent à l'état humide des propriétés qui les rendent idéales comme pansements ophtalmologiques éliminables. Par exemple les lentilles ainsi préparées présentent un faible pouvoir abrasif à l'état humide; lorsqu'on les place sur l'oeil avec un médicament, le mouvement des paupières sur le pansement érode progressivement le pansement si bien qu'il est entraîné par les larmes en 10 à 16 heures. De plus ces lentilles d'hydrogel ou des bandes de pellicule d'hydrogel lorsqu'elles sont saturées d'un médicament pour le traitement du glaucome tel que la pilocarpine ou le Timoptic (composition à base de maléate de timolol) peuvent remplacer les gouttes très visqueuses que l'on dépose sur le globe oculaire.

### Exemple 4

On chauffe à 60°C 400 ml d'un gel limpide constitué d'un mélange de 200 ml d'une solution de gélatine à 10% (ayant un poids moléculaire d'environ 50 000 et une opalescence de 275) et 200 ml de collagène bovin comestible partiellement hydrolysé (ayant un poids moléculaire d'environ 100 000) à 2%. On ajoute à ce mélange en l'agitant de façon douce et régulière, 4 gouttes d'acide chlorhydrique 10 N pour porter le pH à 5,10. Immédiatement après, on ajoute au mélange en agitant énergiquement 8 ml (160 gouttes) de formaline soit environ 12,3% de formaldéhyde par rapport à la protéine, en maintenant la température à 60±5°C.

On doit effectuer la coulée initiale de l'hydrogel naturel préconditionné pour préparer un produit stable à l'eau rapidement après l'addition de l'agent de réticulation, c'est-à-dire avant que la réaction de réticulation ait produit de façon irréversible un gel solide impossible à couler.

### Pellicules ophtalmologiques durables et transplants cornéens

Avec une burette ou une pipette calibrée, on dépose avec précaution 3 gouttes du mélange ci-dessus dans des moules standards en polyméthacrylate de 11 mm de diamètre dans les conditions de température ordinaire. L'épaisseur de la lentille de contact ainsi que ses propriétés de réfraction sont déterminées par les caractéristiques de concavité des moules et le nombre des gouttes qu'on y introduit à une concentration donnée.

On effectue le séchage à l'air, le blanchiment, le lavage, la déshydratation, le lavage et le séchage final à l'air comme décrit en détail dans l'exemple 1.

### Exemple 5

On chauffe à 60°C 400 ml d'un gel limpide constitué de 400 ml d'une solution à 10% de gélatine (ayant un poids moléculaire d'environ 50 000 et une opalescence de 225). On ajoute à ce mélange clair en agitant régulièrement 4 gouttes d'acide chlorhydrique 10 N pour porter le pH à 4,35. Immédiatement après, en agitant énergiquement le mélange, on ajoute 40 gouttes (2 ml) de formaline soit environ 1,85% de formaldéhyde par rapport à la gélatine, en maintenant la température à 60±5°C.

### Hydrogels d'ajustement de la viscosité

On laisse ce produit reposer à la température ordinaire (25°C) pendant au moins 15 minutes en poursuivant l'agitation pour obtenir une homogénéisation maximale du processus de réticulation.

On sèche ensuite l'hydrogel de préférence par séchage instantané par pulvérisation pour le transformer en particules fines. Sinon on peut étaler l'hydrogel sur des plateaux et le sécher lentement à l'air.

Quel que soit le procédé de séchage utilisé, il est nécessaire d'abaisser la teneur en humidité, lors du stade de séchage, en utilisant des températures maximales de 30 à 35°C à la pression atmosphérique, à une valeur ne dépassant pas 10% et de préférence inférieure à 7%.

On plonge ensuite les particules sèches d'hydrogel de polymère protéïque naturel dans une solution aqueuse diluée de peroxyde d'hydrogène classique pour usage domestique (3% en volume) pendant au minimum 1 heure à la température ordinaire (25°C). Ce stade est particulièrement efficace pour éliminer la couleur naturelle jaune brun ou brun clair caractéristique des protéines naturelles de poids moléculaire relativement faible qu'elles soient réticulées ou non.

On soumet ensuite les particules d'hydrogel gonflé à un lavage à fond énergique avec de l'eau de préférence distillée, pour éliminer toutes les petites quantités résiduelles d'agent de réticulation et de peroxyde d'hydrogène.

Après le traitement de lavage à fond ci-dessus, on introduit les particules d'hydrogel gonflé par l'eau dans de l'alcool éthylique du commerce (95% en volume) non dénaturé ou dénaturé où on les laisse séjourner pendant au minimum 2 heures.

A la fin du traitement de déshydratation par trempage dans l'alcool éthylique, on lave les particules d'hydrogel à fond avec de l'eau distillée pour éliminer toute trace d'alcool éthylique résiduel et de composés de dénaturation dans le cas où on utilise de l'alcool éthylique dénaturé. Il est important dans ce stade d'effectuer des lavages poussés, répétés dans de l'eau distillée, de préférence.

On sèche ensuite lentement à l'air les amas d'hydrogel fortement gonflé limpide que l'on a relavés à fond, selon la séquence de séchage précédemment décrite qui consiste soit à les sécher par pulvérisation soit à les sécher à l'air jusqu'à ce que la teneur en humidité résiduelle ne dépasse pas 10% et soit de préférence inférieure à 7%.

Le produit obtenu est particulièrement utile lorsqu'il est gonflé dans des solutions ophtalmologiques aqueuses (par exemple des solutions de chlorhydrate de pilocarpine pour le traitement du glaucome) pour ajuster la viscosité et les propriétés d'écoulement. Surtout la structure interne du réseau fortement gonflé des particules d'hydrogel permet de prolonger l'action des composants médicamenteux auxquels elles sont associées.

## Exemple 6

On chauffe à 60°C 800 ml d'un gel limpide constitué de 600 ml d'une solution à 10% de gélatine et de 200 ml d'un collagène comestible de boeuf partiellement hydrolysé (ayant un poids moléculaire d'environ 100 000) à 1%. On ajoute à ce mélange en agitant doucement et régulièrement 10 gouttes d'acide chlorhydrique 10 N pour porter le pH à 4,80. Immédiatement après, on ajoute au mélange en agitant énergiquement 18 ml de formaline soit environ 10,7% de formaldéhyde par rapport à la protéine, en maintenant la température à $60\pm5°C$.

## Pellicules et pansements pour brûlures et plaies

Immédiatement après avoir mélangé intimement la composition ci-dessus, on la désaère dans un dessicateur sous un vide de 9,48 à $9,8 \cdot 10^4$ Pa pour éliminer les bulles. On coule ensuite le mélange dans des capsules plates en Pyrex dont les dimensions correspondent aux dimensions finales désirées des pellicules. Par exemple pour préparer une pellicule ou un pansement circulaire, on peut utiliser une boîte de Pétri tandis que pour préparer une pellicule rectangulaire, on peut utiliser une capsule rectangulaire de verre.

On laisse l'hydrogel sécher lentement à la température ordinaire (environ 20°C) pendant au moins 24 heures pendant lesquelles la réaction de réticulation atteint pratiquement son achèvement. Selon l'épaisseur désirée de la pellicule, on peut poursuivre le séchage lent à l'air pendant une semaine ou plus jusqu'à ce que la totalité de l'hydrogel ait été séchée avec une humidité résiduelle ne dépassant pas 10% et de préférence inférieure à 7%. Pour réduire la durée de séchage, on peut, après 24 heures, placer le moule contenant la structure façonnée dans une étuve à circulation d'air.

Si on désire produire une pellicule plaquée à un tissu ou incorporée à un tissu avec un tel hydrogel, on dépose régulièrement un tissu à mailles fines dans le moule avant d'y couler l'hydrogel liquide.

On traite ensuite les pellicules coulées selon une succession de traitements comportant un blanchiment avec un agent oxydant ($H_2O_2$) puis un traitement selon des stades de déshydratation avec un solvant organique, etc. comme décrit pour la production des lentilles de contact présentant des propriétés physiques et chimiques remarquables dans l'exemple 1.

### Exemple 7

### Tubes, artères et autres structures

On coule un hydrogel désaéré ayant la composition décrite dans l'exemple 6 dans un moule concentrique pour former un tube (ou une artère) le moule contenant ou non une matrice de tissu.

On laisse la réaction de réticulation s'effectuer à la température ordinaire (25°C) et à 58% d'humidité relative pendant au moins 24 heures jusqu'à ce que la teneur en humidité ne dépasse pas 10%. On retire l'hydrogel solide du moule concentrique pour obtenir une structure tubulaire convenant comme prothèse artérielle.

On soumet ensuite les tubes à un blanchiment, un lavage, une déshydratation, un lavage et un séchage final à l'air comme décrit en détail dans l'exemple 1.

### Exemple 8

### Prothèses osseuses

La matière première utilisée pour produire des prothèses réticulées ou corticales à partir des hydrogels de polymères protéiques naturels stabilisés de l'invention a une composition identique ou très voisine de celle décrite dans l'exemple 6. Une des différences principales est l'emploi d'acide phosphorique plutôt que d'acide chlorhydrique pour ajuster l'acidité de l'hydrogel avant l'addition de l'agent de réticulation.

On disperse intimement des particules ou des cristaux de phosphate de calcium associés ou non à d'autres ions présents naturellement dans les os et les cartilages dans la solution de protéine naturelle non cristalline soluble dans l'eau initiale. Le produit obtenu est essentiellement un mélange physique intime et homogène des divers ingrédients et on peut lui incorporer divers ions pour accroître la dureté du produit avant l'addition des agents de réticulation des protéines décrits dans les exemples précédents.

On peut former le phosphate de calcium par mélange de solutions d'un sel soluble de calcium tel que l'acétate de calcium et d'un phosphate soluble tel que le phosphate de sodium. Dans le cas où l'on ajoute d'autres sels et/ou d'autres ions tels que des ions fluorures ou carbonates, on peut incorporer aux solutions salines lors de la formation du phosphate de calcium, des sels solubles tels que le fluorure de calcium ou le carbonate de sodium. La structure précise des composés de type phosphate de calcium est complexe et dans la présente description le terme »phosphate de calcium« englobe le phosphate dicalcique, le phosphate tricalcique, le phosphate octacalcique, l'hydroxy-apatite, la carbonate-apatite, la chlorapatite, la fluorapatite et leurs mélanges.

Une modification de l'exemple 6 qui s'est révélée souhaitable pour produire des prothèses réticulées extrêmement poreuses, est le remplacement de l'eau utilisée comme solvant de la protéine par une solution aqueuse diluée de peroxyde d'hydrogène, par exemple une solution aqueuse à 1% en volume. Après dissolution de la protéine dans la solution diluée de peroxyde d'hydrogène, on incorpore à la solution de protéine une quantité suffisante d'une suspension aqueuse à 25% en poids de phosphate de calcium pour obtenir des quantités approximativement égales de protéine et de phosphate de calcium. On ajuste ensuite le pH avec de l'acide phosphorique et on ajoute l'agent de réticulation puis on agite énergiquement le mélange en maintenant la température à 60 ± 5° C. L'emploi d'une solution de peroxyde d'hydrogène comme solvant de la protéine provoque la formation de petites bulles dispersées uniformément lors de l'ajustement du pH et de l'addition de l'agent de réticulation ce qui produit, lors de la réticulation et du séchage, une structure de porosité uniforme.

Dans certains cas, il peut être préférable de lyophiliser les compositions d'hydrogel à base de phosphate de calcium obtenues au lieu de les soumettre aux opérations habituelles de séchage à l'air décrites dans les exemples précédents. Dans tous les cas la séquence de séchage initial à une teneur en humidité ne dépassant pas 10% et de préférence de 7% ou moins suivi d'un trempage dans le peroxyde d'hydrogène (à 3% en poids) et d'une déshydratation par un solvant organique comme décrit dans les exemples 1, 2 et 3 demeure souhaitable pour qu'on obtienne une structure osseuse ayant des propriétés de résistance mécanique à l'état humide durables. Par exemple lorsqu'on place ces structures dans de l'eau, elles gonflent mais demeurent cohérentes et ne se désintègrent pas.

Ces produits constituent de nouvelles compositions permettant de produire des structures utiles ressemblant au cartilage, à l'os et à l'ivoire.

### Exemple 9

### Fibres, produits textiles et fils de suture

La composition d'hydrogel de protéine soluble dans l'eau décrite dans l'exemple 6 constitue une matière première appropriée pour la production de nouvelles fibres que l'on peut transformer en de nombreux articles textiles classiques tels que des feuilles, des tissus, des mates, etc.

Avant d'ajouter l'agent de réticulation, on pompe le gel dans une chambre associée à un mécanisme capable d'extruder l'hydrogel à travers des filières pour former des fibres ultrafines ou même des monofils; cet appareillage est couramment utilisé pour produire la rayonne viscose.

On maintient la température du gel à 60 ± 5° C dans la chambre avant de le faire passer dans une pumpe de mélange ou on l'additionne de la quantité appropriée d'agent de réticulation. On extrude les filaments en continu dans de longues chambres de séchage cylindriques verticales où on les sèche à une teneur en humidité ne dépassant pas 10% et de préférence de 7% ou moins puis on les recueille sur des bobines ou sous forme d'écheveaux. Après le stade initial de séchage précédemment décrit, on soumet les filets au traitement d'oxydation (par exemple avec H₂O₂), à la déshydratation par un solvant organique, au lavage poussé final et au séchage final selon des stades opératoires semblables à ceux des exemples précédents.

Ces fibres conviennent à diverses applications dans le domaine médical et le domaine des vêtements. Lorsqu'on les extrude sous forme de filaments de faible diamètre, on peut les utiliser comme fils chirurgicaux pour suture.

**Revendications**

1. Procédé pour préparer une structure tridimensionnelle, caractérisé en ce qu'il consiste à former une solution aqueuse acide d'un polymère protéique, naturel, non cristallin, animal ou végétal ou d'un mélange de tels polymères, ces polymères ayant des poids moléculaires faibles ne dépassant pas 100 000, à chauffer la solution à une température comprise entre 55 et 65°C, à ajuster le pH de la solution entre 3,5 et 5,5, à ajouter à la solution et à lui incorporer, à raison de 0,5% à 15% en poids, un agent de réticulation en maintenant la température de la solution entre 55 et 65°C, à sécher la solution jusqu'à ce que sa teneur en humidité ne dépasse pas 10% à une température maximale de 35°C ou une température équivalente sous vide pour former une structure polymère réticulée, à soumettre cette structure à un lavage poussé par l'eau, à soumettre la structure ainsi lavée à une déshydratation par immersion dans un solvant organique miscible à l'eau, à soumettre la structure à un lavage poussé par l'eau et à sécher à nouveau la structure à une température maximale de 35°C jusqu'à ce que sa teneur en humidité ne dépasse pas 10%.

2. Procédé selon la revendication 1, caractérisé en ce que le polymère protéique est une protéine animale.

3. Procédé selon la revendication 1, caractérisé en ce que le polymère protéique est la gélatine.

4. Procédé selon la revendication 1, caractérisé en ce que le polymère protéique est une protéine végétale.

5. Procédé selon la revendication 1, caractérisé en ce que la solution aqueuse acide du polymère protéique est une solution d'une protéine animale dans l'acide chlorhydrique aqueux ayant un pH compris entre 3,5 et 5,5.

6. Procédé selon la revendication 1, caractérisé en ce que la solution aqueuse acide du polymère protéique est une solution de gélatine dans l'acide chlorhydrique aqueux ayant un pH compris entre 3,5 et 5,5 et en ce que la quantité d'agent de réticulation est comprise entre 0,5 et 15% par rapport au poids de la gélatine.

7. Procédé selon la revendication 1, caractérisé en ce que la solution aqueuse acide du polymère protéique est une solution de gélatine dans l'acide chlorhydrique aqueux ayant un pH compris entre 3,5 et 5,5, l'agent de réticulation est le formaldéhyde et la quantité de formaldéhyde ajoutée est comprise entre 0,5 et 15% par rapport au poids de la gélatine.

8. Procédé selon la revendication 1, caractérisé en ce qu'après séchage de la solution aqueuse acide du polymère contenant l'agent de réticulation pour former une structure polymère réticulée on plonge la structure dans une solution aqueuse d'un agent oxydant avant d'effectuer le lavage initial par l'eau.

9. Procédé selon la revendication 1, caractérisé en ce que le solvant organique miscible à l'eau est l'éthanol.

10. Procédé selon la revendication 1, caractérisé en ce que la solution aqueuse acide du polymère protéique est une solution de gélatine dans l'acide chlorhydrique aqueux ayant un pH compris entre 3,5 et 5,5, l'agent de réticulation est le formaldéhyde, et le quantité de formaldéhyde ajoutée est comprise entre 0,5 et 10% par rapport au poids de la gélatine, en ce qu'après séchage de la solution pour former la structure polymère réticulée, on plonge la structure dans une solution aqueuse de peroxyde d'hydrogène avant d'effectuer le lavage initial par l'eau, et en ce que le solvant organique miscible à l'eau est l'éthanol.

11. Procédé selon la revendication 1, caractérisé en ce que la solution aqueuse acide du polymère protéique est une solution d'une protéine animale dans l'acide chlorhydrique aqueux ayant un pH compris entre 3,5 et 5,5, on incorpore une suspension de phosphate de calcium à la solution acide avant l'addition de l'agent de réticulation, l'agent de réticulation est le formaldehyde, la quantité de formaldéhyde ajoutée est comprise entre 0,5 et 15% par rapport au poids de la protéine animale et le solvant organique miscible à l'eau est l'éthanol.

12. Procédé selon la revendication 1, caractérisé en ce qu'un séchage est poussé jusqu'à ce que la teneur en humidité ne dépasse pas 7%.

13. Procédé selon la revendication 1, caractérisé en ce qu'on termine par un séchage à 120°C.

14. Lentille de contact souple, caractérisée en ce qu'on l'a préparée selon un procédé conforme à l'une quelconque des revendications 1 à 13.

15. Lentille de contact souple obtenue selon un procédé conforme à l'une quelconque des revendications 1 à 13, caractérisée en ce qu'elle est constituée d'un polymère protéique, naturel, non cristallin, animal ou végétal, réticulé, ou d'un mélange de tels polymères, ces polymères ayant un poids moléculaire faible ne dépassant pas 100 000.

## Patentansprüche

1. Verfahren zur Herstellung einer dreidimensionalen Struktur, dadurch gekennzeichnet, daß es darin besteht, daß man eine saure wäßrige Lösung eines natürlichen, nicht kristallinen, tierischen oder pflanzlichen Proteinpolymeren oder einer Mischung solcher Polymerer bildet, wobei diese Polymeren niedrige Molekulargewichte haben, die 100 000 nicht übersteigen, daß man die Lösung auf eine Temperatur zwischen 55 und 65°C erhitzt, das pH der Lösung auf einen Wert zwischen 3,5 und 5,5 einstellt, zur Lösung in einer Menge von 0,5 bis 15 Gewichtsprozent ein Vernetzungsmittel zufügt, und darin einbringt, wobei man die Temperatur der Lösung bei 55 bis 65°C hält, die Lösung trocknet, bis ihr Feuchtigkeitsgehalt 10% nicht übersteigt, und zwar bei einer Maximaltemperatur von 35°C oder einer äquivalenten Temperatur unter Vakuum, um eine vernetzte polymere Struktur zu bilden, diese Struktur einer gründlichen Waschung mit Wasser unterzieht, die so gewaschene Struktur einer Entwässerung durch Eintauchen in ein organisches, mit Wasser mischbares Lösungsmittel unterwirft, die Struktur gründlich mit Wasser wäscht und die Struktur erneut bei einer Temperatur von maximal 35°C bis auf einen Feuchtigkeitsgehalt von nicht mehr als 10% trocknet.

2. Verfahren nach Anspruch 1, dadurch gekennzeichnet, daß das Proteinpolymere ein tierisches Protein ist.

3. Verfahren nach Anspruch 1, dadurch gekennzeichnet, daß das Proteinpolymere Gelatine ist.

4. Verfahren nach Anspruch 1, dadurch gekennzeichnet, daß das Proteinpolymere ein pflanzliches Protein ist.

5. Verfahren nach Anspruch 1, dadurch gekennzeichnet, daß die wäßrige saure Lösung des Proteinpolymeren eine Lösung eines tierischen Proteins in wäßriger Salzsäure mit einem pH zwischen 3,5 und 5,5 ist.

6. Verfahren nach Anspruch 1, dadurch gekennzeichnet, daß die wäßrige saure Lösung des Proteinpolymeren eine Lösung von Gelatine in wäßriger Salzsäure mit einem pH zwischen 3,5 und 5,5 ist, und daß die Menge des Vernetzungsmittels zwischen 0,5 und 15%, bezogen auf das Gewicht der Gelatine, beträgt.

7. Verfahren nach Anspruch 1, dadurch gekennzeichnet, daß die wäßrige saure Lösung des Proteinpolymeren eine Lösung von Gelatine in wäßriger Salzsäure mit einem pH zwischen 3,5 und 5,5, daß Vernetzungsmittel Formaldehyd und die Menge des zugefügten Formaldehyds zwischen 0,5 und 15%, bezogen auf das Gewicht der Gelatine ist.

8. Verfahren nach Anspruch 1, dadurch gekennzeichnet, daß man nach dem Trocknen der wäßrigen sauren Lösung des Polymeren, welche das Vernetzungsmittel enthält, zur Bildung einer vernetzten Polymerstruktur die Struktur in eine wäßrige Lösung eines Oxidationsmittels gibt, bevor man die anfängliche Waschung mit Wasser vornimmt.

9. Verfahren nach Anspruch 1, dadurch gekennzeichnet, daß das organische mit Wasser mischbare Lösungsmittel Ethanol ist.

10. Verfahren nach Anspruch 1, dadurch gekennzeichnet, daß die wäßrige saure Lösung des Proteinpolymeren eine Lösung von Gelatine in wäßriger Salzsäure mit einem pH-Wert von 3,5 bis 5,5, das Vernetzungsmittel Formaldehyd, die Menge des zugesetzten Formaldehyds zwischen 0,5 und 10%, bezogen auf das Gewicht der Gelatine ist, und daß nach dem Trocknen der Lösung zur Bildung der vernetzten polymeren Struktur, man die Struktur in eine wäßrige Lösung von Wasserstoffperoxyd gibt, bevor man das anfängliche Waschen mit Wasser bewirkt, und daß das organische mit Wasser mischbare Lösungsmittel Ethanol ist.

11. Verfahren nach Anspruch 1, dadurch gekennzeichnet, daß die wäßrige saure Lösung des Proteinpolymeren eine Lösung eines tierischen Proteins in wäßriger Salzsäure mit einem pH-Wert zwischen 3,5 und 5,5 ist, daß man eine Suspension von Calciumphosphat in die saure Lösung einbringt, bevor man das Vernetzungsmittel zugibt, daß das Vernetzungsmittel Formaldehyd ist, daß die Menge an zugesetztem Formaldehyd zwischen 0,5 und 15%, bezogen auf das Gewicht des tierischen Proteins ist, und daß das organische mit Wasser mischbare Lösungsmittel Ethanol ist.

12. Verfahren nach Anspruch 1, dadurch gekennzeichnet, daß man die Trocknung soweit treibt, bis der Feuchtigkeitsgehalt 7% nicht übersteigt.

13. Verfahren nach Anspruch 1, dadurch gekennzeichnet, daß man es durch Eintrocknen bis 120°C bis zur Trockne beendet.

14. Biegsame Kontaktlinse, dadurch gekennzeichnet, daß sie gemäß einem Verfahren nach einem der Ansprüche 1 bis 13 hergestellt ist.

15. Biegsame Kontaktlinse, erhalten gemäß einem Verfahren nach einem der Ansprüche 1 bis 13, dadurch gekennzeichnet, daß sie aus einem natürlichen, nicht kristallinen, tierischen oder pflanzlichen vernetzten Proteinpolymeren oder einem Gemisch solcher Polymerer besteht, wobei diese Polymeren ein niedriges Molekulargewicht besitzen, das 100 000 nicht übersteigt.

## Claims

1. Process for the preparation of a three-dimensional structure, characterised in that it consists in forming an acid aqueous solution of a natural, non-crystalline protein polymer of animal or plant origin, or of a mixture of such polymers, these polymers having low molecular weights of not more than 100 000, in heating the solution to a temperature between 55 and 65°C, in adjusting the pH of the solution to between 3.5 and 5.5, in adding a crosslinking agent to the solution and incorporating it therein, in a ratio of

9

0.5% to 15% by weight, the temperature of the solution being kept at between 55 and 65°C, in drying the solution until its moisture content does not exceed 10% at a maximum temperature of 35°C or an equivalent temperature in vacuo, so as to form a crosslinked polymeric structure, in subjecting this structure to thorough washing with water, in subjecting the structure washed in this way to dehydration by immersion in a water-miscible organic solvent, in subjecting the structure to thorough washing with water, and in drying the structure again at a maximum temperature of 35°C, until its moisture content does not exceed 10%.

2. Process according to Claim 1, characterised in that the protein polymer is an animal protein.

3. Process according to Claim 1, characterised in that the protein polymer is gelatine.

4. Process according to Claim 1, characterised in that the protein polymer is a plant protein.

5. Process according to Claim 1, characterised in that the acid aqueous solution of the protein polymer is a solution of an animal protein in aqueous hydrochloric acid having a pH of between 3.5 and 5.5.

6. Process according to Claim 1, characterised in that the acid aqueous solution of the protein polymer is a solution of gelatine in aqueous hydrochloric acid having a pH of between 3.5 and 5.5, and in that the amount of crosslinking agent is between 0.5 and 15%, relative to the weight of the gelatine.

7. Process according to Claim 1, characterised in that the acid aqueous solution of the protein polymer is a solution of gelatine in aqueous hydrochloric acid having a pH of between 3.5 and 5.5, the crosslinking agent is formaldehyde and the amount of formaldehyde added is between 0.5 and 15%, relative to the weight of the gelatine.

8. Process according to Claim 1, characterised in that, after drying of the acid aqueous solution of the polymer, containing the crosslinking agent, so as to form a crosslinked polymeric structure, the structure ist immersed in an aqueous solution of an oxidising agent before the initial washing with water is carried out.

9. Process according to Claim 1, characterised in that the water-miscible organic solvent is ethanol.

10. Process according to Claim 1, characterised in that the acid aqueous solution of the protein polymer is a solution of gelatine in aqueous hydrochloric acid having a pH of between 3.5 and 5.5, the crosslinking agent is formaldehyde and the amount of formaldehyde added is between 0.5 and 10%, relative to the weight of the gelatine, in that, after drying of the solution so as to form the crosslinked polymeric structure, the structure is immersed in an aqueous solution of hydrogen peroxide before the initial washing with water is carried out, and in that the water-miscible organic solvent is ethanol.

11. Process according to Claim 1, characterised in that the acid aqueous solution of the protein polymer is a solution of an animal protein in aqueous hydrochloric acid having a pH of between 3.5 and 5.5, a suspension of calcium phosphate is incorporated into the acid solution before addition of the crosslinking agent, the crosslinking agent ist formaldehyde, the amount of formaldehyde added is between 0.5 and 15%, relative to the weight of the animal protein, and the water-miscible organic solvent is ethanol.

12. Process according to Claim 1, characterised in that drying is pursued until the moisture content does not exceed 7%.

13. Process according to Claim 1, characterised in that the final step is complete drying at 120°C.

14. Flexible contact lens, characterised in that it has been prepared by a process according to any one of Claims 1 to 13.

15. Flexible contact lens obtained by a process according to any one of Claims 1 to 13, characterised in that it consists of a natural, non-crystalline, crosslinked protein polymer of animal or plant origin, or of a mixture of such polymers, these polymers having a low molecular weight of not more than 100 000.